# EUROPEAN PATENT APPLICATION

(11) **EP 1 369 484 A1**
(43) Date of publication of application: **10.12.2003**
(21) Application number: 02705189.5
(22) Date of filing: 14.03.2002
(51) Int. Cl.: C12N 15/52, C12N 15/63, C12N 5/10, A01H 5/00, C12P 7/62

(54) **PLANT SYNTHESIZING COPOLYESTER FROM MONOMER DERIVED FROM SHORT-CHAIN FATTY ACID AND PROCESS FOR PRODUCING POLYESTER**

(30) Priority: 14.03.2001 JP 2001072963
(71) Applicant: Riken, Wako-shi, Saitama 351-0198 (JP)
(72) Inventor: NAKASHITA, Hideo, c/o RIKEN, Wako-shi, Saitama 351-0198 (JP); YAMAGUCHI, Isamu, c/o RIKEN, Wako-shi, Saitama 351-0198 (JP); DOI, Yoshiharu, c/o RIKEN, Wako-shi, Saitama 351-0198 (JP); SUZUKI, Yoshikatsu, c/o RIKEN, Wako-shi, Saitama 351-0198 (JP); KOBAYASHI, Yumiko, c/o RIKEN, Wako-shi, Saitama 351-0198 (JP); SHIMIZU, Toshiyuki, c/o RIKEN, Wako-shi, Saitama 351-0198 (JP)
(74) Representative: Arends, William Gerrit
(86) International application number: JP0202424
(87) International publication number: WO02072837

(57) **Abstract**

This invention provides a plant that can synthesize copolyesters consisting of short chain fatty acid-derived monomers having 4 to 7 carbon atoms and a process for producing copolyesters.

This invention relates to: a fusion gene comprising a gene encoding an enzyme, which synthesizes copolyesters consisting of short chain fatty acid-derived monomers having 4 to 7 carbon atoms and a gene encoding a peroxisome-targeting signal sequence bound thereto; a vector comprising the gene; a transformant comprising the gene; and a process for producing polyesters from the transformant.

SEQUENCE LISTING

<110> RIKEN

<120> Plant that synthesizes copolyesters comprising
      short chain fatty acid-derived monomers and
      process for producing polyesters

<130> PH-1531-PCT

<140>
<141>

<150> JP 2001/72963
<151> 2001-03-14

<160> 19

<170> PatentIn Ver. 2.1

<210> 1
<211> 1785
<212> DNA
<213> Aeromonas caviae

<220>
<221> CDS
<222> (1)..(1785)

<400> 1

atg agc caa cca tct tat ggc ccg ctg ttc gag gcc ctg gcc cac tac    48

Met Ser Gln Pro Ser Tyr Gly Pro Leu Phe Glu Ala Leu Ala His Tyr

1               5                   10                  15

aat gac aag ctg ctg gcc atg gcc aag gcc cag aca gag cgc acc gcc    96

Asn Asp Lys Leu Leu Ala Met Ala Lys Ala Gln Thr Glu Arg Thr Ala

            20                  25                  30

cag gcg ctg ctg cag acc aat ctg gac gat ctg ggc cag gtg ctg gag    144

Gln Ala Leu Leu Gln Thr Asn Leu Asp Asp Leu Gly Gln Val Leu Glu

        35                  40                  45

cag ggc agc cag caa ccc tgg cag ctg atc cag gcc cag atg aac tgg    192

Gln Gly Ser Gln Gln Pro Trp Gln Leu Ile Gln Ala Gln Met Asn Trp

    50                  55                  60

tgg cag gat cag ctc aag ctg atg cag cac acc ctg ctc aaa agc gca    240

Trp Gln Asp Gln Leu Lys Leu Met Gln His Thr Leu Leu Lys Ser Ala

65                  70                  75                  80

ggc cag ccg agc gag ccg gtg atc acc ccg gag cgc agc gat cgc cgc    288

Gly Gln Pro Ser Glu Pro Val Ile Thr Pro Glu Arg Ser Asp Arg Arg

                85                  90                  95

ttc aag gcc gag gcc tgg agc gaa caa ccc atc tat gac tac ctc aag    336

Phe Lys Ala Glu Ala Trp Ser Glu Gln Pro Ile Tyr Asp Tyr Leu Lys

            100                 105                 110

cag tcc tac ctg ctc acc gcc agg cac ctg ctg gcc tcg gtg gat gcc 384
Gln Ser Tyr Leu Leu Thr Ala Arg His Leu Leu Ala Ser Val Asp Ala
        115                 120                 125

ctg gag ggc gtc ccc cag aag agc cgg gag cgg ctg cgt ttc ttc acc 432
Leu Glu Gly Val Pro Gln Lys Ser Arg Glu Arg Leu Arg Phe Phe Thr
    130                 135                 140

cgc cag tac gtc aac gcc atg gcc ccc agc aac ttc ctg gcc acc aac 480
Arg Gln Tyr Val Asn Ala Met Ala Pro Ser Asn Phe Leu Ala Thr Asn
145                 150                 155                 160

ccc gag ctg ctc aag ctg acc ctg gag tcc gac ggc cag aac ctg gtg 528
Pro Glu Leu Leu Lys Leu Thr Leu Glu Ser Asp Gly Gln Asn Leu Val
                165                 170                 175

cgc gga ctg gcc ctc ttg gcc gag gat ctg gag cgc agc gcc gat cag 576
Arg Gly Leu Ala Leu Leu Ala Glu Asp Leu Glu Arg Ser Ala Asp Gln
            180                 185                 190

ctc aac atc cgc ctg acc gac gaa tcc gcc ttc gag ctc ggg cgg gat 624
Leu Asn Ile Arg Leu Thr Asp Glu Ser Ala Phe Glu Leu Gly Arg Asp
        195                 200                 205

ctg gcc ctg acc ccg ggc cgg gtg gtg cag cgc acc gag ctc tat gag 672
Leu Ala Leu Thr Pro Gly Arg Val Val Gln Arg Thr Glu Leu Tyr Glu
    210                 215                 220

ctc att cag tac agc ccg act acc gag acg gtg ggc aag aca cct gtg 720

Leu Ile Gln Tyr Ser Pro Thr Thr Glu Thr Val Gly Lys Thr Pro Val
225                 230                 235                 240

ctg ata gtg ccg ccc ttc atc aac aag tac tac atc atg gac atg cgg    768
Leu Ile Val Pro Pro Phe Ile Asn Lys Tyr Tyr Ile Met Asp Met Arg
                245                 250                 255

ccc cag aac tcc ctg gtc gcc tgg ctg gtc gcc cag ggc cag acg gta    816
Pro Gln Asn Ser Leu Val Ala Trp Leu Val Ala Gln Gly Gln Thr Val
            260                 265                 270

ttc atg atc tcc tgg cgc aac ccg ggc gtg gcc cag gcc caa atc gat    864
Phe Met Ile Ser Trp Arg Asn Pro Gly Val Ala Gln Ala Gln Ile Asp
        275                 280                 285

ctc gac gac tac gtg gtg gat ggc gtc atc gcc gcc ctg gac ggc gtg    912
Leu Asp Asp Tyr Val Val Asp Gly Val Ile Ala Ala Leu Asp Gly Val
    290                 295                 300

gag gcg gcc acc ggc gag cgg gag gtg cac ggc atc ggc tac tgc atc    960
Glu Ala Ala Thr Gly Glu Arg Glu Val His Gly Ile Gly Tyr Cys Ile
305                 310                 315                 320

ggc ggc acc gcc ctg tcg ctc gcc atg ggc tgg ctg gcg gcg cgg cgc    1008
Gly Gly Thr Ala Leu Ser Leu Ala Met Gly Trp Leu Ala Ala Arg Arg
                325                 330                 335

cag aag cag cgg gtg cgc acc gcc acc ctg ttc act acc ctg ctg gac    1056
Gln Lys Gln Arg Val Arg Thr Ala Thr Leu Phe Thr Thr Leu Leu Asp

            340                 345                 350

ttc tcc cag ccc ggg gag ctt ggc atc ttc atc cac gag ccc atc ata 1104
Phe Ser Gln Pro Gly Glu Leu Gly Ile Phe Ile His Glu Pro Ile Ile
        355                 360                 365

gcg gcg ctc gag gcg caa aat gag gcc aag ggc atc atg gac ggg cgc 1152
Ala Ala Leu Glu Ala Gln Asn Glu Ala Lys Gly Ile Met Asp Gly Arg
    370                 375                 380

cag ctg gcg gtc tcc ttc agc ctg ctg cgg gag aac agc ctc tac tgg 1200
Gln Leu Ala Val Ser Phe Ser Leu Leu Arg Glu Asn Ser Leu Tyr Trp
385                 390                 395                 400

aac tac tac atc gac agc tac ctc aag ggt cag agc ccg gtg gcc ttc 1248
Asn Tyr Tyr Ile Asp Ser Tyr Leu Lys Gly Gln Ser Pro Val Ala Phe
                405                 410                 415

gat ctg ctg cac tgg aac agc gac agc acc aat gtg gcg ggc aag acc 1296
Asp Leu Leu His Trp Asn Ser Asp Ser Thr Asn Val Ala Gly Lys Thr
            420                 425                 430

cac aac agc ctg ctg cgc cgt ctc tac ctg gag aac cag ctg gtg aag 1344
His Asn Ser Leu Leu Arg Arg Leu Tyr Leu Glu Asn Gln Leu Val Lys
        435                 440                 445

ggg gag ctc aag atc cgc aac acc cgc atc gat ctc ggc aag gtg aag 1392
Gly Glu Leu Lys Ile Arg Asn Thr Arg Ile Asp Leu Gly Lys Val Lys
    450                 455                 460

acc cct gtg ctg ctg gtg tcg gcg gtg gac gat cac atc gcc ctc tgg 1440
Thr Pro Val Leu Leu Val Ser Ala Val Asp Asp His Ile Ala Leu Trp
465                 470                 475                 480

cag ggc acc tgg cag ggc atg aag ctg ttt ggc ggg gag cag cgc ttc 1488
Gln Gly Thr Trp Gln Gly Met Lys Leu Phe Gly Gly Glu Gln Arg Phe
                485                 490                 495

ctc ctg gcg gag tcc ggc cac atc gcc ggc atc atc aac ccg ccg gcc 1536
Leu Leu Ala Glu Ser Gly His Ile Ala Gly Ile Ile Asn Pro Pro Ala
            500                 505                 510

gcc aac aag tac ggc ttc tgg cac aac ggg gcc gag gcc gag agc ccg 1584
Ala Asn Lys Tyr Gly Phe Trp His Asn Gly Ala Glu Ala Glu Ser Pro
        515                 520                 525

gag agc tgg ctg gca ggg gcg acg cac cag ggc ggc tcc tgg tgg ccc 1632
Glu Ser Trp Leu Ala Gly Ala Thr His Gln Gly Gly Ser Trp Trp Pro
    530                 535                 540

gag atg atg ggc ttt atc cag aac cgt gac gaa ggg tca gag ccc gtc 1680
Glu Met Met Gly Phe Ile Gln Asn Arg Asp Glu Gly Ser Glu Pro Val
545                 550                 555                 560

ccc gcg cgg gtc ccg gag gaa ggg ctg gcc ccc gcc ccc ggc cac tat 1728
Pro Ala Arg Val Pro Glu Glu Gly Leu Ala Pro Ala Pro Gly His Tyr
                565                 570                 575

gtc aag gtg cgg ctc aac ccc gtg ttt gcc tgc cca aca gag gag gac    1776
Val Lys Val Arg Leu Asn Pro Val Phe Ala Cys Pro Thr Glu Glu Asp
            580                 585                 590

gcc gca tga                                                         1785
Ala Ala
        595

<210> 2
<211> 594
<212> PRT
<213> Aeromonas caviae

<400> 2
Met Ser Gln Pro Ser Tyr Gly Pro Leu Phe Glu Ala Leu Ala His Tyr
 1               5                  10                  15
Asn Asp Lys Leu Leu Ala Met Ala Lys Ala Gln Thr Glu Arg Thr Ala
            20                  25                  30
Gln Ala Leu Leu Gln Thr Asn Leu Asp Asp Leu Gly Gln Val Leu Glu
        35                  40                  45
Gln Gly Ser Gln Gln Pro Trp Gln Leu Ile Gln Ala Gln Met Asn Trp
    50                  55                  60
Trp Gln Asp Gln Leu Lys Leu Met Gln His Thr Leu Leu Lys Ser Ala
65                  70                  75                  80
Gly Gln Pro Ser Glu Pro Val Ile Thr Pro Glu Arg Ser Asp Arg Arg
                85                  90                  95
Phe Lys Ala Glu Ala Trp Ser Glu Gln Pro Ile Tyr Asp Tyr Leu Lys
            100                 105                 110

Gln Ser Tyr Leu Leu Thr Ala Arg His Leu Leu Ala Ser Val Asp Ala
        115                 120                 125
Leu Glu Gly Val Pro Gln Lys Ser Arg Glu Arg Leu Arg Phe Phe Thr
    130                 135                 140
Arg Gln Tyr Val Asn Ala Met Ala Pro Ser Asn Phe Leu Ala Thr Asn
145                 150                 155                 160
Pro Glu Leu Leu Lys Leu Thr Leu Glu Ser Asp Gly Gln Asn Leu Val
                165                 170                 175
Arg Gly Leu Ala Leu Leu Ala Glu Asp Leu Glu Arg Ser Ala Asp Gln
            180                 185                 190
Leu Asn Ile Arg Leu Thr Asp Glu Ser Ala Phe Glu Leu Gly Arg Asp
        195                 200                 205
Leu Ala Leu Thr Pro Gly Arg Val Val Gln Arg Thr Glu Leu Tyr Glu
    210                 215                 220
Leu Ile Gln Tyr Ser Pro Thr Thr Glu Thr Val Gly Lys Thr Pro Val
225                 230                 235                 240
Leu Ile Val Pro Pro Phe Ile Asn Lys Tyr Tyr Ile Met Asp Met Arg
                245                 250                 255
Pro Gln Asn Ser Leu Val Ala Trp Leu Val Ala Gln Gly Gln Thr Val
            260                 265                 270
Phe Met Ile Ser Trp Arg Asn Pro Gly Val Ala Gln Ala Gln Ile Asp
        275                 280                 285
Leu Asp Asp Tyr Val Val Asp Gly Val Ile Ala Ala Leu Asp Gly Val
    290                 295                 300
Glu Ala Ala Thr Gly Glu Arg Glu Val His Gly Ile Gly Tyr Cys Ile
305                 310                 315                 320
Gly Gly Thr Ala Leu Ser Leu Ala Met Gly Trp Leu Ala Ala Arg Arg
                325                 330                 335
Gln Lys Gln Arg Val Arg Thr Ala Thr Leu Phe Thr Thr Leu Leu Asp

            340                 345                 350
Phe Ser Gln Pro Gly Glu Leu Gly Ile Phe Ile His Glu Pro Ile Ile
        355                 360                 365
Ala Ala Leu Glu Ala Gln Asn Glu Ala Lys Gly Ile Met Asp Gly Arg
    370                 375                 380
Gln Leu Ala Val Ser Phe Ser Leu Leu Arg Glu Asn Ser Leu Tyr Trp
385                 390                 395                 400
Asn Tyr Tyr Ile Asp Ser Tyr Leu Lys Gly Gln Ser Pro Val Ala Phe
                405                 410                 415
Asp Leu Leu His Trp Asn Ser Asp Ser Thr Asn Val Ala Gly Lys Thr
            420                 425                 430
His Asn Ser Leu Leu Arg Arg Leu Tyr Leu Glu Asn Gln Leu Val Lys
        435                 440                 445
Gly Glu Leu Lys Ile Arg Asn Thr Arg Ile Asp Leu Gly Lys Val Lys
    450                 455                 460
Thr Pro Val Leu Leu Val Ser Ala Val Asp Asp His Ile Ala Leu Trp
465                 470                 475                 480
Gln Gly Thr Trp Gln Gly Met Lys Leu Phe Gly Gly Glu Gln Arg Phe
                485                 490                 495
Leu Leu Ala Glu Ser Gly His Ile Ala Gly Ile Ile Asn Pro Pro Ala
            500                 505                 510
Ala Asn Lys Tyr Gly Phe Trp His Asn Gly Ala Glu Ala Glu Ser Pro
        515                 520                 525
Glu Ser Trp Leu Ala Gly Ala Thr His Gln Gly Gly Ser Trp Trp Pro
    530                 535                 540
Glu Met Met Gly Phe Ile Gln Asn Arg Asp Glu Gly Ser Glu Pro Val
545                 550                 555                 560
Pro Ala Arg Val Pro Glu Glu Gly Leu Ala Pro Ala Pro Gly His Tyr

                565                 570                 575
Val Lys Val Arg Leu Asn Pro Val Phe Ala Cys Pro Thr Glu Glu Asp
            580                 585                 590
Ala Ala

<210> 3
<211> 3187
<212> DNA
<213> Aeromonas caviae

<400> 3
agatctggac cggggtgctg gcctgggcca cgccggcgag ggccagcgcg gagcaaccga 60
gcagcagggc gagaggtttc atcgggattc cttggcagtc tgaatgacgt gccagcctat 120
cagcgcggcg ccggtgcggc gagggcgcgc cggacccagt gcgtcacctc tcgtctgatc 180
cgcctccctc gacgggcgtc gctgacaaaa aaattcaaac agaaattaac atttatgtca 240
tttacaccaa accgcatttg gttgcagaat gctcaaacgt gtgtttgaac agagcaagca 300
acacgtaaac agggatgaca tgcagtaccc gtaagaaggg ccgattggcc cacaacaaca 360
ctgttctgcc gaactggaga ccgatgatga atatggacgt gatcaagagc tttaccgagc 420
agatgcaagg cttcgccgcc cccctcaccc gctacaacca gctgctggcc agcaacatcg 480
aacagctgac ccggttgcag ctggcctccg ccaacgccta cgccgaactg ggcctcaacc 540
agttgcaggc cgtgagcaag gtgcaggaca cccagagcct ggcggccctg ggcacagtgc 600
aactggagac cgccagccag ctctcccgcc agatgctgga tgacatccag aagctgagcg 660
ccctcggcca gcagttcaag gaagagctgg atgtcctgac cgcagacggc atcaagaaaa 720
gcacgggcaa ggcctgataa cccctggctg cccgttcggg cagccacatc tccccatgac 780
tcgacgctac gggctagttc ccgcctcggg tgtgggtgaa ggagagcaca tgagccaacc 840
atcttatggc ccgctgttcg aggccctggc ccactacaat gacaagctgc tggccatggc 900
caaggcccag acagagcgca ccgcccaggc gctgctgcag accaatctgg acgatctggg 960

ccaggtgctg gagcagggca gccagcaacc ctggcagctg atccaggccc agatgaactg 1020
gtggcaggat cagctcaagc tgatgcagca caccctgctc aaaagcgcag gccagccgag 1080
cgagccggtg atcaccccgg agcgcagcga tcgccgcttc aaggccgagg cctggagcga 1140
acaacccatc tatgactacc tcaagcagtc ctacctgctc accgccaggc acctgctggc 1200
ctcggtggat gccctggagg gcgtccccca gaagagccgg gagcggctgc gtttcttcac 1260
ccgccagtac gtcaacgcca tggcccccag caacttcctg gccaccaacc ccgagctgct 1320
caagctgacc ctggagtccg acggccagaa cctggtgcgc ggactggccc tcttggccga 1380
ggatctggag cgcagcgccg atcagctcaa catccgcctg accgacgaat ccgccttcga 1440
gctcgggcgg gatctggccc tgaccccggg ccgggtggtg cagcgcaccg agctctatga 1500
gctcattcag tacagcccga ctaccgagac ggtgggcaag acacctgtgc tgatagtgcc 1560
gcccttcatc aacaagtact acatcatgga catgcggccc cagaactccc tggtcgcctg 1620
gctggtcgcc cagggccaga cggtattcat gatctcctgg cgcaacccgg gcgtggccca 1680
ggcccaaatc gatctcgacg actacgtggt ggatggcgtc atcgccgccc tggacggcgt 1740
ggaggcggcc accggcgagc gggaggtgca cggcatcggc tactgcatcg gcggcaccgc 1800
cctgtcgctc gccatgggct ggctggcggc gcggcgccag aagcagcggg tgcgcaccgc 1860
caccctgttc actaccctgc tggacttctc ccagcccggg gagcttggca tcttcatcca 1920
cgagcccatc atagcggcgc tcgaggcgca aaatgaggcc aagggcatca tggacgggcg 1980
ccagctggcg gtctccttca gcctgctgcg ggagaacagc ctctactgga actactacat 2040
cgacagctac ctcaagggtc agagcccggt ggccttcgat ctgctgcact ggaacagcga 2100
cagcaccaat gtggcgggca agacccacaa cagcctgctg cgccgtctct acctggagaa 2160
ccagctggtg aagggggagc tcaagatccg caacacccgc atcgatctcg gcaaggtgaa 2220
gacccctgtg ctgctggtgt cggcggtgga cgatcacatc gccctctggc agggcacctg 2280
gcagggcatg aagctgtttg gcggggagca gcgcttcctc ctggcggagt ccggccacat 2340
cgccggcatc atcaacccgc cggccgccaa caagtacggc ttctggcaca acggggccga 2400
ggccgagagc ccggagagct ggctggcagg ggcgacgcac cagggcggct cctggtggcc 2460
cgagatgatg ggctttatcc agaaccgtga cgaagggtca gagcccgtcc ccgcgcgggt 2520
cccggaggaa gggctggccc ccgcccccgg ccactatgtc aaggtgcggc tcaaccccgt 2580
gtttgcctgc ccaacagagg aggacgccgc atgagcgcac aatccctgga agtaggccag 2640
aaggcccgtc tcagcaagcg gttcggggcg gcggaggtag ccgccttcgc cgcgctctcg 2700

gaggacttca accccctgca cctggacccg gccttcgccg ccaccacggc gttcgagcgg 2760

cccatagtcc acggcatgct gctcgccagc ctcttctccg ggctgctggg ccagcagttg 2820

ccgggcaagg ggagcatcta tctgggtcaa agcctcagct tcaagctgcc ggtctttgtc 2880

ggggacgagg tgacggccga ggtggaggtg accgcccttc gcgaggacaa gcccatcgcc 2940

accctgacca cccgcatctt cacccaaggc ggcgccctcg ccgtgacggg ggaagccgtg 3000

gtcaagctgc cttaagcacc ggcggcacgc aggcacaatc agcccggccc ctgccgggct 3060

gattgttctc ccccgctccg cttgccccct ttttcggggc aatttggccc aggccctttc 3120

cctgccccgc ctaactgcct aaaatggccg ccctgccgtg taggcattca tccagctaga 3180

ggaattc 3187

<210> 4

<211> 27

<212> DNA

<213> Artificial Sequence

<220>

<223> Description of Artificial Sequence:Synthetic DNA

<400> 4
   ccsccstgga tcaayaagtw ytayatc 27

<210> 5

<211> 27

<212> DNA

<213> Artificial Sequence

<220>

<223> Description of Artificial Sequence:Synthetic DNA

<400> 5
sagccasgcs gtccartcsg gccacca                                          27

<210> 6
<211> 33
<212> DNA
<213> Artificial Sequence

<220>
<223> Description of Artificial Sequence:Synthetic DNA

<400> 6
ctggatcctg gtacctcccc gatcgttcaa aca                                   33

<210> 7
<211> 27
<212> DNA
<213> Artificial Sequence

<220>
<223> Description of Artificial Sequence:Synthetic DNA

<400> 7
ggccagtgaa ttcccgatct agtaaca                                          27

<210> 8

<211> 32

<212> DNA

<213> Artificial Sequence

<220>

<223> Description of Artificial Sequence:Synthetic DNA

<400> 8

ctatggatcc tacatgagcc aaccatctta tg                                    32

<210> 9

<211> 29

<212> DNA

<213> Artificial Sequence

<220>

<223> Description of Artificial Sequence:Synthetic DNA

<400> 9

tctggtacct gcggcgtcct cctctgttg                                    29

<210> 10

<211> 39

<212> DNA

<213> Artificial Sequence

<220>

<223> Description of Artificial Sequence:Synthetic DNA

<400> 10

catacatcat cccagggagc tgtccaggct ctgaggtac 39

<210> 11

<211> 39

<212> DNA

<213> Artificial Sequence

<220>

<223> Description of Artificial Sequence:Synthetic DNA

<400> 11

ctcagagcct ggacagctcc ctgggatgat gtatggtac 39

<210> 12

<211> 39

<212> DNA

<213> Artificial Sequence

<220>

<223> Description of Artificial Sequence:Synthetic DNA

<400> 12

catacatcat cccagggagc tggccaggct ctgaggtac                    39

<210> 13
<211> 39
<212> DNA
<213> Artificial Sequence

<220>
<223> Description of Artificial Sequence:Synthetic DNA

<400> 13
ctcagagcct ggccagctcc ctgggatgat gtatggtac                    39

<210> 14
<211> 10
<212> PRT
<213> Cucurbita sp.

<400> 14
Ile His His Pro Arg Glu Leu Ser Arg Leu
 1               5                  10

<210> 15
<211> 10
<212> PRT
<213> Spinacia oleracea

<400> 15
Ile His His Pro Arg Glu Leu Ala Arg Leu
1               5                   10

<210> 16
<211> 30
<212> DNA
<213> Artificial Sequence

<220>
<223> Description of Artificial Sequence:Synthetic DNA

<400> 16
atacatcatc ccagggagct gtccaggctc                                        30

<210> 17
<211> 30
<212> DNA
<213> Artificial Sequence

<220>
<223> Description of Artificial Sequence:Synthetic DNA

<400> 17
atacatcatc ccagggagct ggccaggctc                                        30

<210> 18

<211> 5

<212> PRT

<213> Cucurbita sp.

<400> 18

Glu Leu Ser Arg Leu
1               5

<210> 19

<211> 5

<212> PRT

<213> Spinacia oleracea

<400> 19

Glu Leu Ala Arg Leu
1               5

## Description

### Technical Field

The present invention relates to a plant that can synthesize copolyesters comprising short chain fatty acid-derived monomers and a process for producing copolyesters.

### Prior Art

Polyesters, which are biologically synthesized by microorganisms (for example, poly-3-hydroxyalkanoic acid), have thermoplasticity and are biodegradable plastics ranging from hard to viscoelastic rubber-like plastics.

Recently, binary copolyesters comprising 3-hydroxybutyrate (3HB) and 3-hydroxyhexanoate (3HH), i.e., P(3HB-co-3HH), and a process for producing the same, have been researched and developed (for example, JP Patent Publication (Kokai) Nos. 5-93049 A (1993) and 7-265065 A (1995)). In the processes for producing the P(3HB-co-3HH) copolymers described in these publications, the P(3HB-co-3HH) copolymers are produced by fermentation from oleic acid or olive oil using *Aeromonas caviae* isolated from soil. The degree of crystallization of the P(3HB-co-3HH) copolymers that were produced by fermentation for the purpose of energy storage is lowered as the proportion of the 3HH unit increases. Accordingly, they can be flexible polymeric materials and can be processed into strong threads or transparent, soft films that are excellent in thermal stability and workability (Y. Doi, S. Kitamura, H. Abe, Macromolecules 28, 4822-4823 (1995)). In addition, polyesters that were produced from microorganisms are useful from the viewpoint of environmental conservation, since they are biodegradable. Accordingly, polyesters that were produced and accumulated from microorganisms can be used for various applications.

When producing polyesters using microorganisms, however, specific equipment such as a culture apparatus or medium is necessary. Since petroleum energy must be consumed in order to activate the equipment, the production cost is disadvantageously increased.

Accordingly, it is desired to develop a means for mass-producing polyesters having the aforementioned properties at low cost without relying on a means for culturing microorganisms.

### Disclosure of the Invention

A system for producing polyesters in plants is constituted using *Arabidopsis thaliana*, rapeseed, maize, and tobacco. Polyesters that have been heretofore produced were either homopolyesters comprising 3-hydroxybutanoic acid or copolyesters comprising medium chain fatty acid-derived monomers having 6 to 12 carbon atoms. The utility values of these polyesters are not very high from a practical standpoint because of their physicochemical properties. Copolyesters comprising monomers having 4 to 5 carbon atoms were also produced, although excess ammonia was generated when producing monomers having 5 carbon atoms from threonine. This adversely affected the growth of plants. Thus, it has been desired to develop a system for synthesizing large amounts of copolyesters comprising short chain fatty acid-derived monomers (4 to 7 carbon atoms) that have excellent thermoplastic properties, etc. and high utility values.

An object of the present invention is to provide a recombinant expression vector comprising a modified gene in which a polyester synthase gene that can polymerize monomers having approximately 4 to 7 carbon atoms are localized in the peroxisome containing a glyoxysome, a transgenic plant that can synthesize copolyesters comprising short chain fatty acid-derived monomers, and a process for producing copolyesters comprising short chain fatty acid-derived monomers.

In order to attain the above object, the present inventors have focused on the presence of short chain fatty acid-derived monomers having 4 to 7 carbon atoms in the peroxisome, considered that polyesters of interest can be obtained if a polyester synthase, which can copolymerize short chain fatty acids having 4 to 7 carbon atoms, is expressed to be localized in the peroxisome, and conducted concentrates studies. In their studies, they have introduced a fusion gene that is prepared by modifying a gene encoding a polyester synthase which can polymerize monomers having approximately 4 to 7 carbon atoms such that the expressed proteins are localized in the peroxisome or a vector that comprises the fusion gene incorporated therein in a plant and expressed it therein. As a result, they have succeeded in mass-producing useful copolyesters from plants. This has led to the completion of the present invention.

More specifically, the present invention is as follows:
(1) a fusion gene comprising a gene encoding an enzyme, which synthesizes copolyesters consisting of short chain fatty acid-derived monomers having 4 to 7 carbon atoms, and a gene encoding a peroxisome-targeting signal sequence bound thereto;
(2) the fusion gene according to (1) above, wherein the gene encoding an enzyme, which synthesizes copolyesters, encodes the protein according to the following (a) or (b):
   (a) a protein consisting of the amino acid sequence as shown in SEQ ID NO: 2; or
   (b) a protein consisting of an amino acid sequence with deletion, substitution, or addition of at least one amino acid in the amino acid sequence as shown in SEQ ID NO: 2 and having polyester synthase activity;
(3) the fusion gene according to (1) above, wherein the gene encoding an enzyme, which synthesizes copolyesters, comprises the DNA according to the following (c) or (d):
   (c) DNA consisting of the nucleotide sequence as shown in SEQ ID NO: 1; or
   (d) DNA hybridizing with DNA consisting of a nucleotide sequence complementary to the DNA consisting of the nucleotide sequence of (c) under stringent conditions and encoding a protein having polyester synthase activity;
(4) the fusion gene according to any one of (1) to (3) above, wherein the gene encoding a peroxisome-targeting signal sequence comprises the DNA according to the following (e) or (f):
   (e) DNA consisting of the nucleotide sequence as shown in SEQ ID NO: 16 or 17; or
   (f) DNA hybridizing with DNA consisting of a nucleotide sequence complementary to the DNA consisting of the nucleotide sequence according to (e) under stringent conditions and encoding a peptide having peroxisome-targeting signal sequence activity;
(5) the fusion gene according to any one of (1) to (3) above, wherein the gene encoding a peroxisome-targeting signal sequence encodes the peptide according to the following (g) or (h):
   (g) a peptide consisting of the amino acid sequence as shown in SEQ ID NO: 14 or 15; or
   (h) a peptide consisting of an amino acid sequence with deletion, substitution, or addition of at least one amino acid in the amino acid sequence as shown in SEQ ID NO: 14 or 15 and having peroxisome-targeting signal sequence activity;
(6) a recombinant vector comprising the fusion gene according to any one of (1) to (5);
(7) a transformant comprising the recombinant vector according to (6) above;
(8) the transformant according to (6) above, which is a plant body, plant organ, plant tissue, or cultured plant cell;
(9) a transformant comprising the fusion gene according to any one of (1) to (5) above introduced therein, which is a plant expressing the fusion gene so that the copolyester synthases are localized in the peroxisome;
(10) the transformant according to (8) or (9) above, which is a plant belonging to a genus selected from the group consisting of *Solanaceae, Gramineae, Malvaceae, Brassicaceae, Compositae, Pedaliaceae, Oleaceae, Myrtaceae, Rosaceae, Camelliaceae, Leguminosae, Palmae, Sterculiaceae,* and *Rubiaceae;*
(11) the transformant according to (10) above, wherein the plant belonging to *Solanaceae* is tobacco;
(12) the transformant according to (10) above, wherein the plant belonging to *Brassicaceae* is *Arabidopsis thaliana;*
(13) a process for producing polyesters, wherein the transformant according to any one of (7) to (12) above is cultured or cultivated, and polyesters are obtained from the resulting culture product or cultivation product; and
(14) the production process according to (13) above, wherein polyesters are copolymers of 3-hydroxyalkanoic acid represented by formula I: 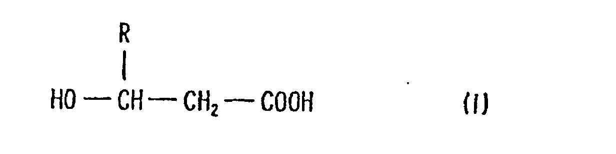
wherein R represents a hydrogen atom or an alkyl group having 1 to 4 carbon atoms.

The present invention is hereafter described in detail.

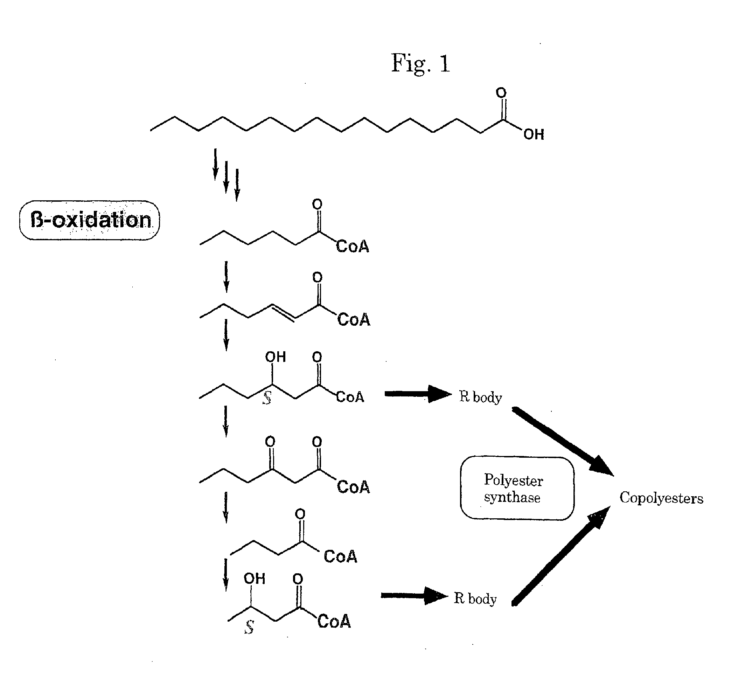

In the present invention, a gene encoding a polyester synthase (this may be referred to as a "polyester synthase gene") and a gene encoding a peroxisome-targeting signal sequence (a transitional signal sequence) are incorporated in the same expression vector, the resulting recombinant expression vector is introduced into a plant to prepare a transformant (a transgenic plant), and copolyesters comprising short chain fatty acid-derived 3-hydroxyalkanoic acid monomers having approximately 4 to 7 carbon atoms, which have thermoplastic, biodegradable, elastic, and other properties, are mass-produced from the transformant. Fig. 1 shows a pathway for synthesizing copolyesters in the peroxisome.

The term "peroxisome" used herein is a concept that includes "glyoxysome."

### 1. Cloning of polyester synthase gene

### (1) Cloning of polyester synthase gene

In the present invention, a polyester synthase gene to be incorporated into a recombinant expression vector comprises a gene encoding an enzyme that synthesizes copolyesters from short chain fatty acid monomers having 4 to 7 carbon atoms. Examples of such a gene include polyester polymerase genes of *Nocardia coralina, Ralstonia eutropha,* and *Phodospirillum rabrum.* An example of preferable gene is the one encoding a polyhydroxyalkanoic acid synthase that is isolated from a microbial cell belonging to the genus *Aeromonas* (this may be hereafter referred to as a "phaC gene"). The polyhydroxyalkanoic acid synthase has a catalytic function of generating poly-3-hydroxyalkanoic acid using 3-hydroxybutyryl-CoA as a substrate. In addition, this enzyme has a catalytic function of generating poly-3-hydroxyalkanoic acid using at least 3-hydroxypentanoyl-CoA or 3-hydroxyhexanoyl-CoA as a substrate.

At the outset, chromosome DNA is prepared from a cell having the phaC gene, for example, *Aeromonas caviae* FA440. Chromosome DNA can be prepared by a conventional process. For example, *Aeromonas caviae* FA440 is cultured in the LB medium, and chromosome DNA is then prepared by the hexadecyl trimethyl ammonium bromide method (Current Protocols in Molecular Biology, vol. 1, pp. 2.4.3, John Wiley & Sons, 1994) or the like.

The DNA obtained by the above method is partially degraded with a suitable restriction enzyme (for example, *Sau*3AI, *Bam*HI, or *Bgl*II) and then treated with alkaline phosphatase, followed by dephosphorylation of a DNA fragment. The resultant is ligated to a vector that was cleaved with a restriction enzyme (for example, *Bam*HI*,* or *Bgl*II) to prepare a library.

A phage or plasmid that is capable of autonomous multiplication in a host microorganism is used as a vector. Examples of a phage vector include EMBL3, M13, and λgt11. Examples of a plasmid vector include pBR322, pUC18, and pBluescript II (Stratagene). In addition to a vector that is capable of autonomous multiplication in two or more host microorganisms such as *E. coli* and *Bacillus brevis,* various shuttle vectors can be further used. Such vectors are cleaved with the aforementioned restriction enzyme, and thus, fragments thereof can be obtained.

A known DNA ligase is used to ligate a DNA fragment to a vector fragment. Thereafter, the DNA fragment and the vector fragment are annealed and then ligated to prepare a recombinant vector.

A recombinant vector can be introduced into a host microorganism by a conventional method. For example, when a host microorganism is *E. coli,* the calcium method (Lederberg, E. M. et al., J. Bacteriol. 119, 1072 (1974)) or electroporation (Current Protocols in Molecular Biology, vol. 1, pp. 1.8.4, 1994) can be employed. When a host microorganism is a phage DNA, the *in vitro* packaging method (Current Protocols in Molecular Biology, vol. 1, pp. 5.7.1, 1994) or the like can be employed. In the present invention, a kit for *in vitro* packaging (e.g., Gigapack II; Stratagene) is used.

A probe for obtaining a DNA fragment comprising the phaC gene is then prepared. Several amino acid sequences of the polyester synthase are already known (e.g., Peoples, O.P. and Sinskey, A. J, J. Biol. Chem., 264, 15293 (1989); Huisman, G. W. et al., J. Biol. Chem., 266, 2191 (1991); Pieper, U. et al., FEMS Microbiol. Lett., 96, 73 (1992)). Two of the conserved regions in these amino acid sequences are selected, and nucleotide sequences encoding the same are deduced to design oligonucleotides. Examples of these oligonucleotides include, but are not limited to, two types of oligonucleotides represented by 5'-CC(C/G)CC(C/G)TGGATCAA(T/C)AAGT(T/A)(T/C)TA(T/C)ATC-3' (SEQ ID NO: 4) and 5'-(G/C)AGCCA(G/C)GC(G/C)GTCCA(A/G)TC(G/C)GGCCACCA-3' (SEQ ID NO: 5).

The polymerase chain reaction (PCR) is carried out using these oligonucleotides as primers and chromosome DNA of *Aeromonas caviae* as a template (Molecular Cloning, vol. 2, pp. 14.2, 1989). The polyester synthase gene is partially amplified by PCR.

Subsequently, this partially amplified fragment is labeled with a suitable reagent, and colony hybridization is carried out using the aforementioned chromosome DNA library (Current Protocols in Molecular Biology, vol.1, pp. 6.0.3, 1994).

In accordance with the alkali method, a plasmid is collected from the *E. coli* that was screened for by colony hybridization (Current Protocols in Molecular Biology, vol. 1, pp. 1.6.1, 1994). Thus, a DNA fragment comprising a polyester synthase gene is obtained.

Nucleotide sequencing of the aforementioned DNA fragment can be carried out by a conventional process, for example, the Sanger method (Molecular Cloning, vol. 2, pp. 13.3, 1989). In general, a nucleotide sequence automatic analyzer, for example, 373A DNA Sequencer (Applied Biosystems) is used.

SEQ ID NO: 3 shows the full sequence of the phaC gene, SEQ ID NO: 1 shows a nucleotide sequence of an open reading frame (ORF) of the phaC gene, and SEQ ID NO: 2 shows an amino acid sequence encoded by the ORF. As long as a polypeptide having the amino acid sequence exhibits polyester synthase activity, there may be some variations such as deletion, substitution, or addition of several amino acids. The gene of the present invention has a nucleotide sequence encoding the amino acid sequence as shown in SEQ ID NO: 2 or an amino acid sequence having variation. In addition, the gene includes a degenerate isomer encoding the same polypeptides, which are different from each other only in their degenerate codons.

For example, at least one, preferably about 1 to 10, and more preferably 1 to 5 amino acids may be deleted from the amino acid sequence as shown in SEQ ID NO: 2. At least one, preferably about 1 to 10, and more preferably 1 to 5 amino acids may be added to the amino acid sequence as shown in SEQ ID NO: 2. Alternatively, at least one, preferably about 1 to 10, and more preferably 1 to 5 amino acids in the amino acid sequence as shown in SEQ ID NO: 2 may be substituted with other amino acids.

An example of such an amino acid sequence with deletion, substitution, or addition of one or several amino acids in the amino acid sequence as shown in SEQ ID NO: 2 is a sequence that is at least 80%, preferably at least 90%, more preferably at least 95%, and particularly preferably at least 97%, at least 98%, or at least 99% homologous to the amino acid sequence as shown in SEQ ID NO: 2 when calculated using BLAST.

Such an amino acid sequence with deletion, substitution, or addition of one or several amino acids in the amino acid sequence as shown in SEQ ID NO: 1 or 2 is substantially identical to the amino acid sequence as shown in SEQ ID NO: 1 or 2.

The aforementioned variations, such as deletion, can be induced by conventional site-directed mutagenesis (Current Protocols in Molecular Biology, vol. 1, pp. 8.1.1, 1994) or a commercially available kit (LA PCR *in vitro* Mutagenesis Series Kit, Takara).

The phaC gene includes a DNA that hybridizes with another DNA comprising a nucleotide sequence complementary thereto under stringent conditions and encodes a protein having polyester synthase activity as well as a DNA comprising a nucleotide sequence as shown in SEQ ID NO: 1 or 3. Stringent conditions involve, for example, a sodium concentration of 500 to 1,000 mM, preferably 700 mM, and a temperature of 50 to 70°C, preferably 65°C.

Once the nucleotide sequence of the phaC gene is defined, a polyester synthase gene can be then obtained either by chemical synthesis, PCR in which the nucleotide sequences at the 5'- and 3'-terminuses of the gene are used as primers and the genomic DNA (SEQ ID NO: 1 or 3) is used as a template, or hybridization using a DNA fragment having a nucleotide sequence of the phaC gene as a probe.

The phaC gene obtained from *Aeromonas caviae* FA440 is introduced into *Alcaligenes eutrophus* (also known as *Ralstonia eutropha)* (name: *Alcaligens eutrophus* AC32), and was deposited at the International Patent Organism Depositary of the National Institute of Advanced Industrial Science and Technology (Tsukuba Central 6, 1-1-1 Higashi, Tsukuba, Ibaraki, Japan) as of August 12, 1996 (an original deposit) under the accession number FERM BP-6038.

### 2. Preparation of recombinant expression vector and transformant

With the addition of a suitable signal sequence to enzyme proteins encoded by a polyester synthase gene such as the phaC gene, the enzyme proteins that are encoded by a polyester synthase gene expressed in plants can be localized in the peroxisome. A fusion protein that was prepared by adding a suitable signal sequence to the enzyme proteins encoded by the polyester synthase gene is produced by a fusion gene comprising the polyester synthase gene and a gene encoding a suitable signal sequence bound thereto.

Transitional signal sequences for transitioning a malate synthase (e.g., a pumpkin-derived malate synthase) or glycolate oxidase (e.g., a spinach-derived glycolate oxidase) to the peroxisome can be used as signal sequences.

Signal sequences of hydroxypyruvate reductase, isocitrate lyase, or uricase can be also used.

The amino acid sequences of the peroxisome-targeting signal sequences are as follows:

(I) IHHPRELSRL (SEQ ID NO: 14)

(II) IHHPRELARL (SEQ ID NO: 15).

Even if at least 1, 2, or 3 amino acids therein are substituted with other amino acids, at least 1, 2, or 3 amino acids are added thereto, or at least 1, 2, or 3 amino acids are deleted therefrom, the sequence can be used as long as it has the activity of a peroxisome-targeting signal sequence, i.e., as long as it functions as a transitional signal to peroxisome. For example, 5 amino acid sequences ELSRL (SEQ ID NO: 18) or ELARL (SEQ ID NO: 19) on the C-terminal side of the above amino acid sequence can function as peroxisome-targeting signals, and 3 amino acid sequences SRL or ARL on the C-terminal side can function as peroxisome-targeting signals. Amino acid sequences SKL, SRM, or PRL and a sequence in which 3 amino acids at the C-terminus of the amino acid sequence as shown in SEQ ID NO: 14, 15, 18, or 19 have been substituted with the aforementioned sequences can also function as peroxisome-targeting signals. Further, a sequence comprising 3 amino acids represented by (S, A, or P)-(K or R)-(L or M) and the amino acid sequence as shown in SEQ ID NO: 14, 15, 18, or 19 in which 3 amino acids at its C-terminus have been substituted with the above sequence can also function as peroxisome-targeting signals.

Examples of sequences of genes encoding a signal sequence as shown in SEQ ID NO: 14 or 15 include the following:

(III) ATACATCATCCCAGGGAGCTGTCCAGGCTC (SEQ ID NO: 16)

(IV) ATACATCATCCCAGGGAGCTGGCCAGGCTC (SEQ ID NO: 17).

A sequence of the gene encoding a signal sequence can be prepared from a synthetic DNA strand. It can also be prepared by cloning from a genomic DNA of a suitable plant.

A gene encoding a signal sequence can be fused with a polyester synthase gene by a conventional gene recombination technique. In such a case, a suitable restriction site can be introduced. A signal sequence may be added to the C-terminal side or N-terminal side of the enzyme protein encoded by the polyester synthase gene. In this case, a stop codon should be prevented from appearing between the polyester synthase gene and a gene encoding a signal sequence. The distance between the gene encoding the signal sequence and the polyester synthase gene is not particularly limited, and a linker may be contained therebetween. In order to allow the fusion protein having both the polyester synthase activity and the signal sequence activity to be translated, the open reading frames of the both genes should be aligned with each other. Further, there may be a nucleotide sequence in which a specific amino acid is contained in a linker portion, so that the polyester synthase and the signal sequence are enzymatically cleaved in the peroxisome.

For example, a DNA strand, which encodes a signal sequence fusing with the polyester synthase gene, can be prepared in the following manner.

When the following sequences (V) and (VI) are prepared from synthetic DNA strands, the synthetic DNA strands of the following combinations are subjected to thermal denaturation, followed by annealing.

(V)
SRL-U; CATACATCATCCCAGGGAGCTGTCCAGGCTCTGAGGTAC (SEQ ID NO: 10)

SRL-L; CTCAGAGCCTGGACAGCTCCCTGGGATGATGTATGGTAC (SEQ ID NO: 11)

(VI)
ARL-U; CATACATCATCCCAGGGAGCTGGCCAGGCTCTGAGGTAC (SEQ ID NO: 12)

ARL-L; CTCAGAGCCTGGCCAGCTCCCTGGGATGATGTATGGTAC (SEQ ID NO: 13)

If some nucleotides in these nucleotide sequences are different, a sequence can be still used as long as it is identical to the amino acid sequence of the signal peptide when translated into amino acid. An example thereof is a DNA, which hybridizes with these DNAs under stringent conditions and encodes a protein having activity of the peroxisome-targeting signal sequence. Stringent conditions involve, for example, a sodium concentration of 500 to 1,000 mM, preferably 700 mM, and a temperature of 50 to 70°C, preferably 65°C.

### 3. Preparation of recombinant expression vector

The polyester synthase gene such as the phaC gene prepared as described in 1. above was introduced into a suitable vector with a promoter, thereby constructing the recombinant expression vector of the present invention.

Vectors for inserting the polyester synthase gene and the signal sequence are not particularly limited as long as they can be replicated in a host. Examples thereof include a plasmid DNA and a phage DNA.

A plasmid DNA (a binary vector) can be prepared from E. *coli* or *Agrobacterium* by alkali extraction (Birnboim, H. C. & Doly, J. (1979), Nucleic acid Res 7: 1513) or the like. Examples of a binary vector are pBI121 and pBI101. Commercially available plasmids, for example, pUC118 (Takara Shuzo Co., Ltd.), pUC119 (Takara Shuzo Co., Ltd.), pBluescript SK+ (Stratagene), pGEM-T (Promega), or pCR2.1 (Invitrogen) can be also used.

Examples of a phage DNA include M13mp18, M13mp19, and M13tv18.

In order to insert a promoter into a vector, for example, a purified polyester synthase gene and a signal sequence are first cleaved with a suitable restriction enzyme and inserted into a restriction enzyme site or multicloning site of a suitable vector DNA, thereby ligating it to the vector. Examples of a promoter include a cauliflower mosaic virus 35S promoter, an actin promoter, and a promoter of the nopaline synthase gene.

The polyester synthase gene and the signal sequence to be expressed need to be incorporated into a vector in such a manner that the functions of these genes are exhibited. Accordingly, in addition to the promoter and the gene, a terminator, a drug-resistant gene, or the like can be incorporated into the recombinant expression vector of the present invention. In this case, an example of a terminator is that of a nopaline synthase gene. An example of an enhancer is a tobacco mosaic virus Ω sequence. Examples of a drug-resistant gene include a kanamycin-resistant gene, a hygromycin-resistant gene, a bialaphos-resistant gene, and a blasticidin S-resistant gene.

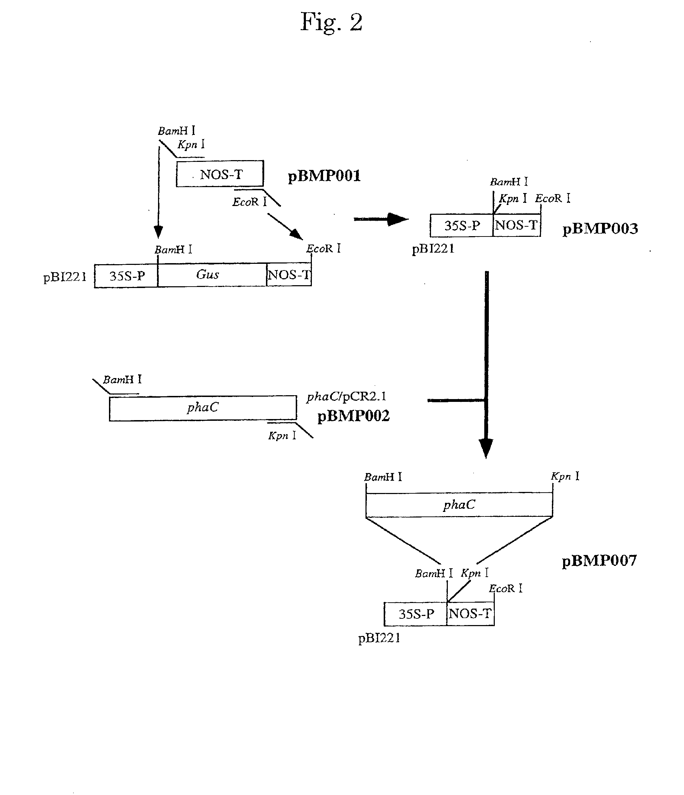

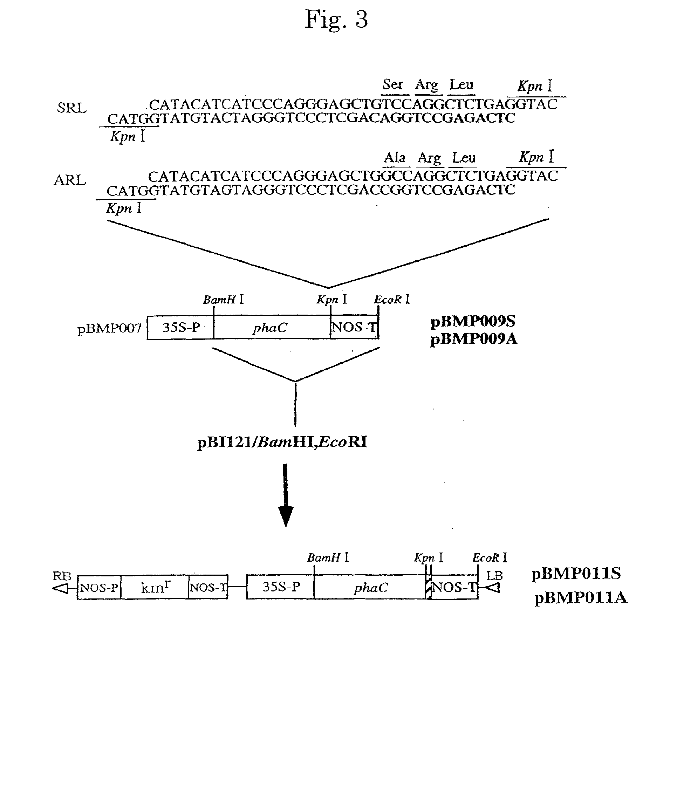

Specifically, the phaC gene, which comprises a peroxisome-targeting signal sequence (malate synthase-derived SRL or glycolate oxidase-derived ARL), is ligated downstream of the cauliflower mosaic virus 35S promoter (35S-P) added thereto, and the terminator (Nos-T) of the nopaline synthase gene is ligated further downstream (this is referred to as a "phaC gene-signal sequence cassette") as shown in Figs. 2 and 3. Also, a cassette comprising a kanamycin-resistant gene (Km^{r}) ligated between the promoter and the terminator of the nopaline synthase gene is prepared. The kanamycin-resistant gene cassette and the phaC gene-signal sequence cassette are ligated in that order, thereby obtaining the recombinant expression vector (pBMP011S and pBMP011A) of the present invention (Fig. 3).

### 4. Preparation of transformant

The transformant (transgenic plant) of the present invention can be obtained by introducing the recombinant vector of the present invention into a host in such a manner that a gene of interest can be expressed.

The term "host" used herein refers to any of a whole plant, plant organs (e.g., leaves, flower petals, stems, roots, or seeds), plant tissues (e.g., epidermises, phloems, parenchymas, xylems, fibrovascular bundles, palisade tissues, or spongy tissues), or cultured cells of plants. Examples of plants used in the transformation include those belonging to *Solanaceae, Gramineae, Malvaceae, Brassicaceae, Compositae, Pedaliaceae, Oleaceae, Myrtaceae, Rosaceae, Leguminosae, Palmae,* or *Rubiaceae.* Examples of plants belonging to these genera include the following, although are not limited thereto. Any plant can be selected as long as it can generate polyesters upon the introduction of the recombinant expression vector of the present invention therein.
*Solanaceae:* tobacco *(Nicotiana tabacum)* and potato *(Solanum tuberosum)*
*Gramineae:* maize *(Zea mays)* and rice *(Oryza sativa)*
*Malvaceae:* cotton *(Gossypium hirsutum),* okra *(Abelmoscus esculentum),* and kenaf *(Hibiscus cannabinus)*
*Brassicaceae: Arabidopsis thaliana* and *Brassica napus*
*Compositae:* sunflower *(Helianthus annuus)* and mum *(Crysanthimum indicum)*
*Pedaliaceae:* sesame *(Sesame indica)* and castor seed *(Ricinus communis)*
*Oleaceae:* olive *(Olea europaea)*
*Myrtaceae:* eucalyptus *(Eucalyptus globulus)* and guava *(Psidium guava)*
*Rosaceae:* rose *(Rosa sinnis)*
*Camelliaceae:* Camellia *(Camellia japonica)*
*Leguminosae:* Japanese milk-vetch *(Astragalus sinicus)* and soybean *(Glycine max)*
*Palmae:* coconut *(Cocos nucifera)*
*Sterculiaceae:* cocoa *(Theobroma cacao)*
*Rubiaceae:* coffee plant *(Coffea arabica)*

When a plant body, plant organ, or plant tissue is used as a host, transformation can be carried out by introducing the recombinant expression vector of the present invention into a slice of the collected plant by the *Agrobacterium* method. Alternatively, the vector can be introduced into a protoplast by electroporation, or can be introduced into a plant organ or plant tissue by the particle bombardment method.

Tumor tissues, shoots, capillary roots, or the like resulting from the transformation can be used as such for cell culture, tissue culture, or organ culture. Also, with the use of any conventional method of culturing plant tissues, plant bodies can be regenerated by, for example, the administration of suitably concentrated plant hormones.

When cultured plant cells are used as hosts, the recombinant expression vector of the present invention is introduced into cultured cells by means of electroporation, the *Agrobacterium* binary vector method, or the like, and cell culture, tissue culture, or organ culture is then carried out in the same manner as described above, thereby obtaining transgenic plant bodies.

### 5. Production of polyesters

Polyesters can be produced by culturing or cultivating a transformant comprising a recombinant expression vector obtained as described in 4. above.

When a transformant is a plant cell or plant tissue, culture can be carried out using a common medium for plant culture, for example, MS basal medium (Murashige, T. & Skoog, F. (1962), Physiol. Plant. 15: 473), LS basal medium (Linsmaier, E. M. &Skoog, F. (1965), Physiol. Plant. 18: 100), or protoplast culture medium (modified LS medium). Culture may be carried out by a common solid-culture method, although a liquid-culture method is preferable.

A cell, tissue, or organ is injected into the above medium in an amount of 0.5 to 40 g fresh weight/l NAA, 2,4-D, BA, kinetin, or the like is suitably added if necessary, and culture is then conducted. At the initiation of culture, the pH level of the medium is adjusted to between 5.6 and 5.8, and culture is conducted generally at 25 to 30°C and preferably at around 25°C while agitating at 20 to 120 rpm for 1 to 8 weeks.

When a transformant is a plant body, the plant body can be cultivated in a field, green house, or the like. Alternatively, a transformant can be hydroponically cultivated.

After the completion of culture, cultured polyesters can be collected by a general purification means for polyesters. Depending on the conditions of cultured cells or tissues and plant organs or bodies, polyesters are purified after being washed with 50% ethanol or methanol.

When polyesters are collected from cultured cells or tissues, cells are destroyed by cytolysis using enzymes such as cellulase or pectinase, ultrasonic breaking, abrasion, or the like. Subsequently, insoluble substances are removed by filtration, centrifugation, or the like to obtain a crude solution of polyester.

In order to further purify polyesters from the crude solution of polyester, common purification techniques can be employed in addition to solvent extraction with chloroform, ethyl acetate, or the like. For example, gas chromatography, liquid chromatography, mass analysis, or NMR can be performed singly or in suitable combinations.

When polyesters are collected from plant organs or bodies, an extract of the polyesters is prepared by ultrasonic breaking, abrasion, or the like, extraction is performed with chloroform, and polyesters can be then obtained in the same manner as that of the aforementioned purification technique.

The thus obtained polyesters are copolymers of 3-hydroxyalkanoic acid (degree of polymerization: 1 to 1,000,000) represented by formula I: 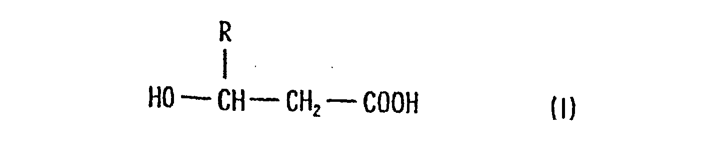 wherein R represents a hydrogen atom or an alkyl group having 1 to 4 carbon atoms.

For example, the copolymers have a structure of poly-3-hydroxybutanoic acid represented by the following formula II: 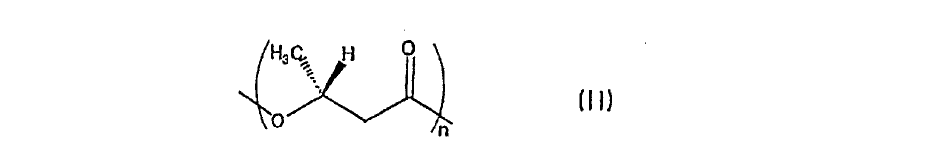 wherein n is an integer between 1 and 1,000,000 or that of polyhydroxyalkanoic acid represented by the following formula III: 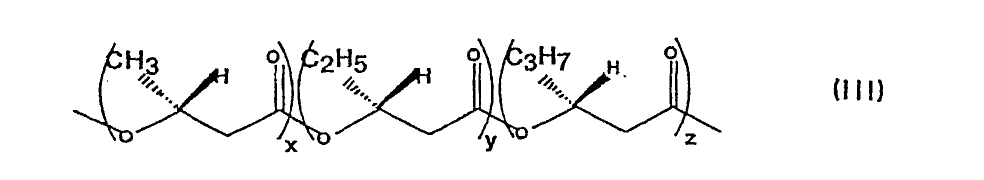 wherein x is an integer between 0 and 1,000,000; y is an integer between 0 and 1,000,000; and z is an integer between 0 and 1,000,000.

### Brief Description of Drawings

Fig. 1 shows a pathway for synthesizing copolyesters using a β oxidation pathway of fatty acid in the peroxisome.
Fig. 2 shows constructions of plasmids pBMP001, pBMP002, pBMP003, and pBMP007.
Fig. 3 shows constructions of plasmids pBMP009S, pBMP009A, pBMP011S, and pBMP011A.
Fig. 4 is an electrophoretic photograph showing the results of phbC mRNA detection by RT-PCR, wherein an arrow on the phaC gene indicates an amplified region, M indicates a size marker (ϕ x 174/HaeIII), SRL indicates a plant having pBMP011S introduced therein, and ARL indicates a plant having pBMP011 A introduced therein.
Fig. 5 is an electrophoretic photograph showing the results of PHA synthase detection by Western blotting, wherein P indicates haC, N indicates Samsun NN, A25Tₗ indicates a self-reproduced progeny of the ARL tobacco into which pBMP011A has been introduced, A1 and A4 indicate the ARL tobacco into which pBMP011A has been introduced, and S1 indicates the SRL *Arabidopsis thaliana* into which pBMP011S has been introduced.
Fig. 6A is an electrophoretic photograph showing the results of the introduced polyester synthase gene (*Xho*I-treated) detection by Southern blotting, wherein C indicates a non-transformant, SRL indicates tobacco into which pBMP011S has been introduced, ARL indicates tobacco into which pBMP011A has been introduced, and numbers indicate lines of individual transformants.
Fig. 6B is an electrophoretic photograph showing the results of the introduced polyester synthase gene (*Bgl*II-treated) detection by Southern blotting, wherein C indicates a non-transformant, SRL indicates tobacco into which pBMP011S has been introduced, ARL indicates tobacco into which pBMP011A has been introduced, and numbers indicate lines of individual transformants.
Fig. 7A shows the results of gas chromatography analysis on polyesters produced from tobacco (SRL).
Fig. 7B shows the results of mass spectrum analysis on polyesters produced from tobacco (SRL).
Fig. 8A shows the results of gas chromatography analysis on polyesters produced from *Arabidopsis thaliana* (SRL).
Fig. 8B shows the results of mass spectrum analysis on polyesters produced from *Arabidopsis thaliana* (SRL).

### Description of Sequence Listing

SEQ ID NO: 4 to 13: synthetic DNA
SEQ ID NO: 14: malate synthase peroxisome-targeting signal sequence
SEQ ID NO: 15: glycolate oxidase peroxisome-targeting signal sequence
SEQ ID NO: 16: synthetic DNA
SEQ ID NO: 17: synthetic DNA
SEQ ID NO: 18: malate synthase peroxisome-targeting signal sequence
SEQ ID NO: 19: glycolate oxidase peroxisome-targeting signal sequence

### Best Modes for Carrying out the Invention

The present invention is hereafter described in more detail with reference to the examples, although the technical scope of the present invention is not limited to these examples.

The following examples describe the production of copolyesters in the peroxisome of a transgenic plant using a peroxisome-targeting signal derived from a malate synthase (SRL) or glycolate oxidase (ARL).

### [Example 1] Construction of recombinant expression vector

(1) The *Bam*HI and *Kpn*I sites were introduced into the 5'-domain of the nopaline synthase (NOS) terminator. The NOS-T domain was amplified by LA-PCR using pBI221 as a template and primers NOST-U and NOST-L prepared by adding restriction enzyme sites to specific sequences. Further, the *Bam*HI and *Kpn*I sites were added to the 5'-side and *Eco*RI was added to the C-terminal side. The PCR product was cloned into the TA cloning vector pCR2.1 to confirm this was the correct sequence, and the confirmed sequence was designated as pBMP001. Primer sequences are as follows. NOST-U; CTGGATCCTGGTACCTCCCCGATCGTTCAAACA (SEQ ID NO: 6) NOST-L; GGCCAGTGAATTCCCGATCTAGTAACA (SEQ ID NO: 7)
   PCR was performed for 30 PCR cycles of 98°C for 20 seconds and 55°C for 5 minutes.
   Fig. 2 shows the construction of pBMP001.
   The *Bam*HI*-Eco*RI domain containing a pBMP001 NOS terminator was replaced with the *Bam*HI*-Eco*RI domain of pBI221 to prepare pBMP003.
   Fig. 2 shows the construction of pBMP003.
(2) Preparation of pBMP002
   Primers, Cper-U and Cper-LK, the sequences of which are indicated below and which were prepared by adding restriction enzyme sites to sequences specific to the phaC gene, were used to amplify the phaC domain by LA-PCR with the further addition of a *Bam*HI site to the N-terminal side and *Kpn*I to the C-terminal side. The PCR product was cloned into the TA cloning vector pCR2.1 to prepare pBMP002. Cper-U; CTATGGATCCTACATGAGCCAACCATCTTATG (SEQ ID NO: 8) Cper-LK; TCTGGTACCTGCGGCGTCCTCCTCTGTTG (SEQ ID NO: 9)
   PCR was performed for 30 PCR cycles of 98°C for 20 seconds and 55°C for 5 minutes.
   Fig. 2 shows the construction of pBMP002.
(3) Preparation of pBMP007
   The *Bam*HI*-Kpn*I domain containing phaC of pBMP002 was inserted into the *Bam*HI*-Kpn*I domain of pBM003 to prepare pBMP007.
   Fig. 2 shows the construction of pBMP007.
(4) Addition of a peroxisome-targeting signal sequence to the C-terminal side of phaC
   The following combination of synthetic DNA strands SRL-U and SRL-L and that of ARL-U and ARL-L were subjected to thermal denaturation at 95°C for 5 minutes, followed by annealing at 68°C for 10 minutes. Thus, gene fragments SRL and ARL encoding the peroxisome-targeting signal sequence were prepared. The resultants were introduced into the *Kpn*I site of pBMP007 using the *Kpn*I site on both terminuses of these fragments. Whether or not SRL-U or ARL-U was inserted in the same direction as that of the phaC gene was confirmed, and sequences into which gene fragments SRL and ARL had been inserted were designated as pBMP009S and pBMP009A, respectively.
   The amino acid sequences of the peroxisome-targeting signal are as shown below. SRL: IHHPRELSRL (SEQ ID NO: 14) ARL: IHHPRELARL (SEQ ID NO: 15) SRL-U; CATACATCATCCCAGGGAGCTGTCCAGGCTCTGAGGTAC (SEQ ID NO: 10) SRL-L; CTCAGAGCCTGGACAGCTCCCTGGGATGATGTATGGTAC (SEQ ID NO: 11) ARL-U; CATACATCATCCCAGGGAGCTGGCCAGGCTCTGAGGTAC (SEQ ID NO: 12) ARL-L; CTCAGAGCCTGGCCAGCTCCCTGGGATGATGTATGGTAC (SEQ ID NO: 13)
   Fig. 3 shows the constructions of pBMP009S and pBMP009A.
(5) Preparation of pBMP011S and pBMP011A
   The BamHI-EcoRI domain containing phaC and NOS terminator of pBMP009S and pBMP009A was replaced with the *Bam*HI*-Eco*RI domain of pBI121 to prepare pBMP011S and pBMP011A.
   Fig. 3 shows the constructions of pBMP011S and pBMP011A.

### [Example 2] Transformation of tobacco

(1) Introduction of plant expression vector into plant
   A plant expression vector that was constructed to localize the introduced genes in the peroxisome was introduced into tobacco by the leaf disk method. The same expression vector was also introduced into *Arabidopsis thaliana* by the floral dip method.
(2) Transformation of *Agrobacterium*
   Plant expression vectors pBMP011S and pBMP011A were introduced into *Agrobacterium tumefaciens* LBA4404 by the direct method. The introduction of genes was confirmed by colony PCR.
(3) Transformation
   With the use of the *Agrobacterium* into which plant expression vectors pBMP011S and pBMP011A had been introduced, tobacco (variety: Samsun NN, Xanthi nc) was transformed by the leaf disk method. Redifferentiated plant bodies were subjected to selection twice employing the kanamycin-resistance as an indication, and resistant plants were determined to be transformants.
(4) Acquisition of transgenic plant
   In the system using Samsun NN, 98 SRL individuals and 125 ARL individuals were found to be kanamycin-resistant in the secondary selection. In the system using Xanthi nc, 24 SRL individuals and 30 ARL individuals were found to be kanamycin-resistant in the secondary selection.
(5) Detection of expression of introduced genes
   Accumulation of mRNA of the introduced genes and the PHA synthase, which is an expression product of phaC, in the kanamycin-resistant individuals were detected. Total RNA was extracted from leaves of kanamycin-resistant plants. cDNA was synthesized from the extracted total RNA, and RT-PCR was conducted. Since the amplification of the full length phaC was difficult, PCR was carried out for 30 PCR cycles of thermal denaturation at 95°C for 30 seconds, lowering temperature for 3 minutes, annealing at 55°C for 1 minute, and elongation at 72°C for 2 minutes using a sequence-specific primer.
(6) Confirmation of transformation by RT-PCR
   Accumulation of mRNA was confirmed in 65 SRL individuals and 85 ARL individuals in the system using Samsun NN. Accumulation of mRNA was confirmed in 16 SRL individuals and 18 ARL individuals in the system using Xahthi nc.

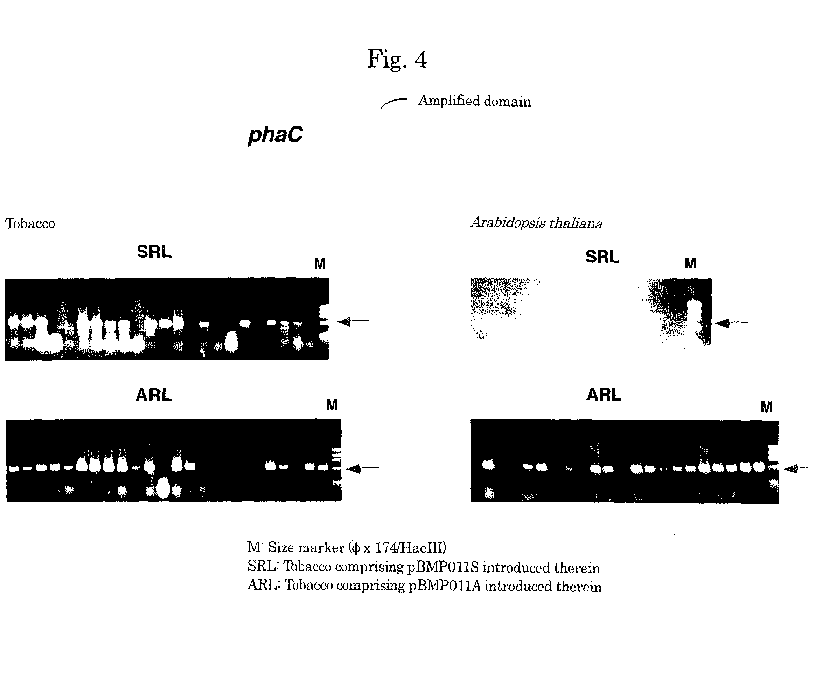

   Fig. 4 shows the detection of a DNA fragment amplified by RT-PCR.
   The following experiment was conducted on some of the kanamycin-resistant plants.

### [Example 3] Confirmation of a polyester synthase in a transformant

(1) Detection of expressed protein by Western blotting
   In the transformants that were found to have accumulated mRNA of the introduced genes by RT-PCR, a cell extract prepared from leaves was separated in 7% SDS-PAGE and then transferred on a cellulose membrane. Western blotting was performed using an anti-PHA synthase polyclonal antibody as a primary antibody and a horseradish peroxidase-labeled anti-rabbit antibody as a secondary antibody to detect by chemoluminescence using peroxidase.
(2) Confirmation of accumulation of expressed protein
   Accumulation of PhaC proteins was confirmed in an ARL individual (A25T1) in the system using Samsun NN. Accumulation of PhaC proteins was confirmed in ARL individuals (A1 and A4) and an SRL individual (S1) in the system using *Arabidopsis thaliana.*

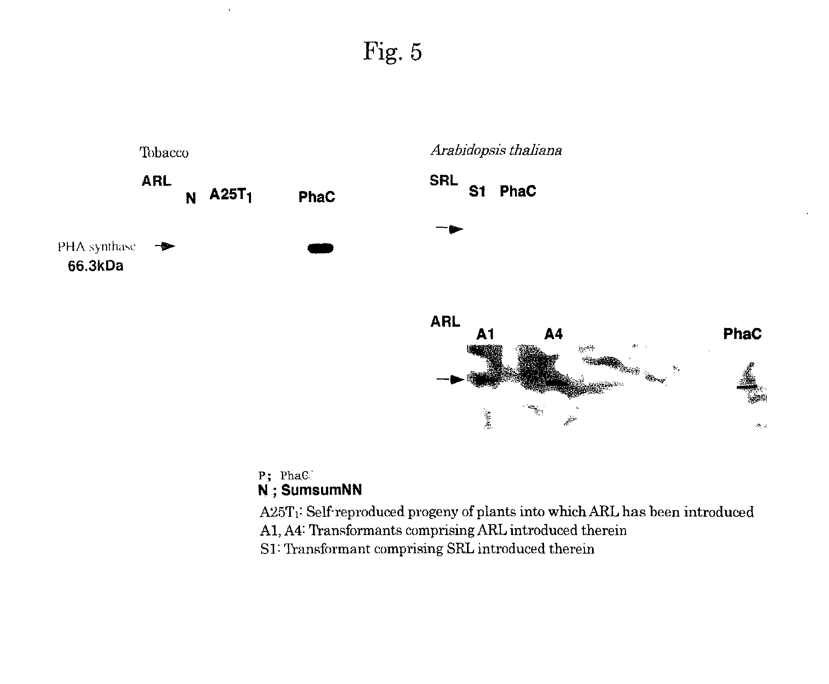

   Fig. 5 shows the result of detecting proteins expressed in a transgenic tobacco by Western blotting.
(3) Detection of introduced genes in a chromosome by Southern blotting
   A chromosome DNA of the plant to be transformed was extracted, treated with *BglII* or *Xho*I*,* separated in 0.8% agarose gel electrophoresis, and transferred on a nylon membrane to immobilize it thereon by ultraviolet irradiation. A part of the domain (496 bp) on the 5'-terminal side of PhaC was labeled with α-32P dCTP to use it as a probe. Hybridization was carried out at 65°C for 16 hours, followed by washing and detection. The results are as shown below.
   A single band was detected in the A8 individual treated with *Bgl*II or *Xho*I.
   Simultaneously with this individual, 4 individuals (S3, S4, S19, and S20) were subjected to detection, and many bands were found therein.

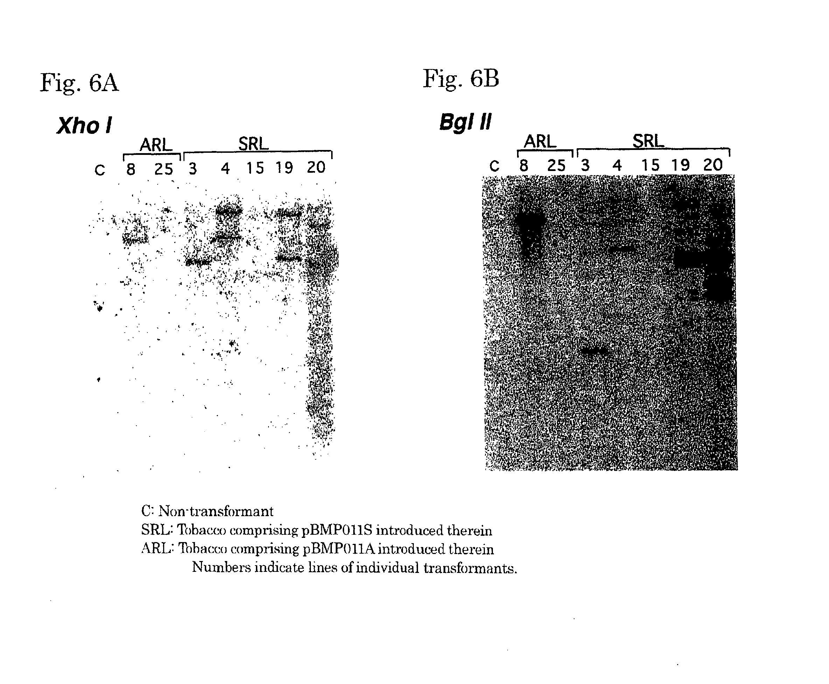

   Fig. 6 shows the results of detecting the genes introduced in the transgenic tobacco by Southern blotting.

### [Example 4] Production of polyesters from transformant

(1) Confirmation of polyester productivity by GC-MS analysis
   Plant leaves were washed for 24 hours with 100% methanol using the Soxhlet extractor, followed by extraction with chloroform for 48 hours. The chloroform-extracted fraction was concentrated, and a 10-fold amount of methanol was added thereto for recrystallization. Ethanol (0.85 ml) and 0.1 ml of HCl were added to 0.25 ml of chloroform solution comprising the crystal dissolved therein, followed by hermetical sealing. Thereafter, the reaction was allowed to proceed at 100°C for 4 hours for ethanolysis. The reaction solution was cooled and then washed with 1.5 ml of aqueous solution of 0.9N NaCl·0.65N NaOH. The chloroform fraction was then recovered and subjected to GC-MS analysis. Assay was carried out under conditions of initial column temperature at 45°C and temperature increments from 45°C to 117°C at 5°C/min and from 117°C to 300°C at 40°C/min. 3-Hydroxyisoheptanoic acid was used as a standard substance, and quantification was carried out by preparing the calibration curves of ethyl 3-hydroxybutyrate (ethyl 3HB) and ethyl 3-hydroxyhexanoate (ethyl 3HHx).
(2) Production of polyesters from plants comprising polyester synthases localized in the peroxisome
   Ethyl 3- hydroxybutyrate, ethyl 3-hydroxypentanoate, and ethyl 3-hydroxyhexanoate were detected in the transformants of SRL and ARL in which accumulation of mRNA was confirmed. This revealed that the resulting transformants produced copolyesters comprising C4 to C6 short chain fatty acid-derived monomers.
   According to the total ion mass chromatogram, the ratio of these thee types of monomers are C4:C5:C6 = 1:0.1 to 1.2:0.05 to 0.3. The deviation could be larger depending on cultivation conditions or the like.
   As a result of quantification using 3-hydroxyisoheptanoic acid as a standard sample, the C4:C6 ratio was 1:0.1 to 0.8. The deviation could be larger depending on cultivation conditions or the like.
   As a result of quantification of C4 and C6, the amount produced was found to be approximately 9 to 101 ppm or more based on the dry weight of tobacco leaves. The deviation could be larger depending on cultivation conditions or the like.

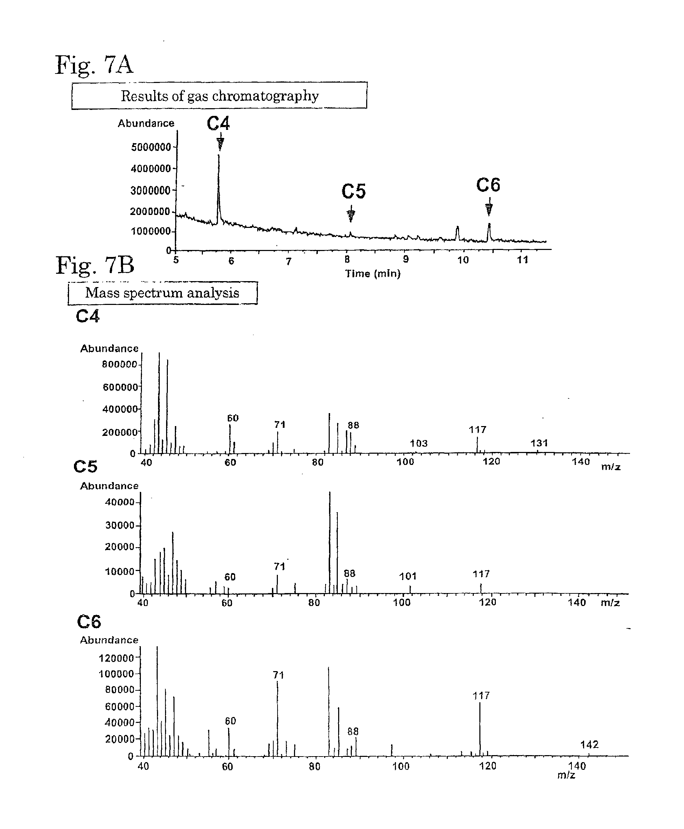

   Fig. 7 shows the results of gas chromatography analysis on copolyesters produced by transgenic tobacco and the results of mass spectrum analysis on the peak.

### [Example 5] Transformation of Arabidopsis thaliana

(I) Transformation *of Arabidopsis thaliana*
   *Arabidopsis thaliana* was infected with *Agrobacterium* by the floral dip method. Analysis was carried out by RT-PCR and Western blotting as with the case of the tobacco. According to the analysis by RT-PCR, 59 ARL individuals and 35 SRL individuals were found to be kanamycin-resistant in the system using *Arabidopsis thaliana* (Fig. 4). According to the Western blotting analysis, accumulation of PhaC proteins were observed in ARL individuals (A1 and A4) and an SRL individual (S1) in the system *using Arabidopsis thaliana* (Fig. 5).

The PHB productivity was as follows.

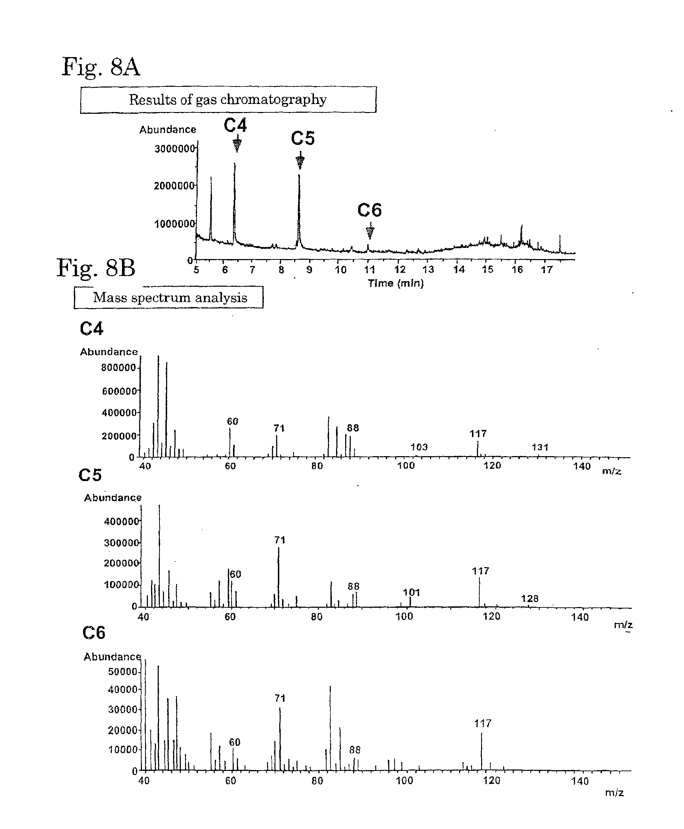

Both ethyl 3HB and ethyl 3HHx were detected in the transformants of SRL and ARL in which accumulation of mRNA was observed (Fig. 8).

According to total ion mass chromatogram, the ratio of these monomers are C4:C5:C6 = 1:0.1 to 0.25:0.05 to 0.3. The deviation could be larger depending on cultivation conditions or the like.

As a result of quantification using 3-hydroxyisoheptanoic acid as a standard sample, the ratio was approximately C4:C6 = 1:0.1 to 1.1. The deviation could be larger depending on cultivation conditions or the like.

The amount produced was approximately 13 to 53 ppm or more based on the dry weight of *Arabidopsis thaliana.* The deviation could be larger depending on cultivation conditions or the like.

### Industrial Applicability

The present invention provides a recombinant expression vector comprising a polyester synthase gene and a peroxisome-targeting signal sequence, a transformant that can synthesize copolyesters comprising short chain fatty acid-derived monomers, and a process for producing polyesters. The transformant of the present invention can mass-produce polyesters at low cost without petroleum resources. In addition, the resulting polyesters are thermoplastic, biodegradable, and elastic, and thus, are very useful due to their wide range of applications.

## Claims

1. A fusion gene comprising a gene encoding an enzyme, which synthesizes copolyesters consisting of short chain fatty acid-derived monomers having 4 to 7 carbon atoms, and a gene encoding a peroxisome-targeting signal sequence bound thereto.

2. The fusion gene according to claim 1, wherein the gene encoding an enzyme, which synthesizes copolyesters, encodes the protein according to the following (a) or (b):
(a) a protein consisting of the amino acid sequence as shown in SEQ ID NO: 2; or
(b) a protein consisting of an amino acid sequence with deletion, substitution, or addition of at least one amino acid in the amino acid sequence as shown in SEQ ID NO: 2 and having polyester synthase activity.

3. The fusion gene according to claim 1, wherein the gene encoding an enzyme, which synthesizes copolyesters, comprises the DNA according to the following (c) or (d):
(c) DNA consisting of the nucleotide sequence as shown in SEQ ID NO: 1; or
(d) DNA hybridizing with DNA consisting of a nucleotide sequence complementary to the DNA consisting of the nucleotide sequence of (c) under stringent conditions and encoding a protein having polyester synthase activity.

4. The fusion gene according to any one of claims 1 to 3, wherein the gene encoding a peroxisome-targeting signal sequence comprises the DNA according to the following (e) or (f):
(e) DNA consisting of the nucleotide sequence as shown in SEQ ID NO: 16 or 17; or
(f) DNA hybridizing with DNA consisting of a nucleotide sequence complementary to the DNA consisting of the nucleotide sequence according to (e) under stringent conditions and encoding a peptide having peroxisome-targeting signal sequence activity.

5. The fusion gene according to any one of claims 1 to 3, wherein the gene encoding a peroxisome-targeting signal sequence encodes the peptide according to the following (g) or (h):
(g) a peptide consisting of the amino acid sequence as shown in SEQ ID NO: 14 or 15; or
(h) a peptide consisting of an amino acid sequence with deletion, substitution, or addition of at least one amino acid in the amino acid sequence as shown in SEQ ID NO: 14 or 15 and having peroxisome-targeting signal sequence activity.

6. A recombinant vector comprising the fusion gene according to any one of claims 1 to 5.

7. A transformant comprising the recombinant vector according to claim 6.

8. The transformant according to claim 7, which is a plant body, plant organ, plant tissue, or cultured plant cell.

9. A transformant comprising the fusion gene according to any one of claims 1 to 5 introduced therein, which is a plant expressing the fusion gene so that the copolyester synthases are localized in the peroxisome.

10. The transformant according to claim 8 or 9, which is a plant belonging to a genus selected from the group consisting of *Solanaceae, Gramineae, Malvaceae, Brassicaceae, Compositae, Pedaliaceae, Oleaceae, Myrtaceae, Rosaceae, Camelliaceae, Leguminosae, Palmae, Sterculiaceae,* and *Rubiaceae.*

11. The transformant according to claim 10, wherein the plant belonging to *Solanaceae* is tobacco.

12. The transformant according to claim 10, wherein the plant belonging to *Brassicaceae* is *Arabidopsis thaliana.*

13. A process for producing polyesters, wherein the transformant according to any one of claims 7 to 12 is cultured or cultivated, and polyesters are obtained from the resulting culture product or cultivation product.

14. The production process according to claim 13, wherein polyesters are copolymers of 3-hydroxyalkanoic acid represented by formula I: 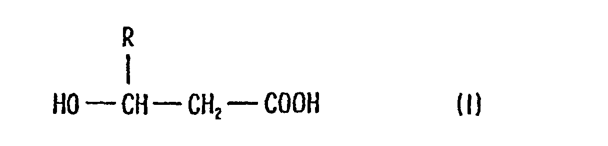 wherein R represents a hydrogen atom or an alkyl group having 1 to 4 carbon atoms.
